# FASCICULE DE BREVET EUROPEEN

(11) **EP 1 203 762 B1**
(45) Date de publication et mention de la délivrance du brevet: **02.07.2008**
(21) Numéro de dépôt: 02290042.7
(22) Date de dépôt: 13.07.1998
(51) Int. Cl.: C07C 211/63, C07C 217/84, A61K 8/14, A61K 8/49

(54) **Novelles bases d'oxydation cationiques, leur utilisation pour la teinture d'oxydation des fibres keratiniques, compositions tinctoriales de procédes de teinture**
Kationische Oxidatinsbasen, deren Verwendung zur Oxydationsfärbung von keratinischen Fasern, Färbezusammensetzungen und Verfahren
Cationic oxidation bases, their use for oxidation dyeing of keratin fibres, dyeing compositions and dyeing methods

(30) Priorité: 16.07.1997 FR 9709027
(43) Date de publication de la demande: 08.05.2002
(62) Demande divisionnaire de: 98938744.4
(73) Titulaire: L'ORÉAL, 75008 Paris (FR)
(72) Inventeur: Genet, Alain, 93600 Aulnay sous Bois (FR); Lagrange, Alain, 77700 Coupvray (FR)
(74) Mandataire: Miszputen, Laurent

(56) Documents cités:
- EP-A- 0 673 926
- DE-B- 1 292 784
- GB-A- 2 018 453
- US-A- 5 139 532

## Description

L'invention a pour objet de nouvelles bases d'oxydation monobenzéniques comportant au moins un groupement cationique Z, Z étant choisi parmi des chaînes aliphatiques quaternisées et des chaînes aliphatiques contenant au moins un cycle saturé quaternisé, leur utilisation pour la teinture d'oxydation des fibres kératiniques, les compositions tinctoriales les contenant, ainsi que les procédés de teinture d'oxydation les mettant en oeuvre.

Il est connu de teindre les fibres kératiniques et en particulier les cheveux humains avec des compositions tinctoriales contenant des précurseurs de colorant d'oxydation, en particulier des ortho ou paraphénylènediamines, des ortho ou paraaminophénols, des composés hétérocycliques tels que des dérivés de diaminopyrazole, appelés généralement bases d'oxydation. Les précurseurs de colorants d'oxydation, ou bases d'oxydation, sont des composés incolores ou faiblement colorés qui, associés à des produits oxydants, peuvent donner naissance par un processus de condensation oxydative à des composés colorés et colorants.

On sait également que l'on peut faire varier les nuances obtenues avec ces bases d'oxydation en les associant à des coupleurs ou modificateurs de coloration, ces derniers étant choisis notamment parmi les métadiamines aromatiques, les métaaminophénols, les métadiphénols et certains composés hétérocycliques.

La variété des molécules mises en jeu au niveau des bases d'oxydation et des coupleurs, permet l'obtention d'une riche palette de couleurs.

La coloration dite "permanente" obtenue grâce à ces colorants d'oxydation, doit par ailleurs satisfaire un certain nombre d'exigences. Ainsi, elle doit être sans inconvénient sur le plan toxicologique, elle doit permettre d'obtenir des nuances dans l'intensité souhaitée et présenter une bonne tenue face aux agents extérieurs (lumière, intempéries, lavage, ondulation permanente, transpiration, frottements).

Les colorants doivent également permettre de couvrir les cheveux blancs, et être enfin les moins sélectifs possible, c'est à dire permettre d'obtenir des écarts de coloration les plus faibles possible tout au long d'une même fibre kératinique, qui peut être en effet différemment sensibilisée (i.e. abîmée) entre sa pointe et sa racine.

Il a déjà été proposé, notamment dans le brevet US 5,139,532, d'utiliser certains dérivés cationiques de paraphénylènediamines, à savoir plus précisément des paraphénylènediamines dont un des groupements amino est monosubstitué par une chaîne aliphatique quaternisée, pour la teinture d'oxydation des fibres kératiniques dans des nuances intenses et plus rouges que celles obtenues habituellement en mettant en oeuvre des paraphénylènediamines classiques, c'est à dire ne portant pas de groupement cationique. Toutefois, l'utilisation des paraphénylènediamines décrites dans ce brevet antérieur ne permet pas d'obtenir une riche palette de couleurs et, de plus, les colorations obtenues ne donnent pas toujours entière satisfaction du point de vue de leur résistance vis à vis des diverses agressions que peuvent subir les cheveux (action de la lumière, de la transpiration, des shampooings, etc...).

DE-A-1292784 décrit aussi des colorants directs nitro azoïques. EP-A- 0673926 décrit des composés para-phényènediamine soufrés en position 5 et nitro en position 2 du cycle benzénique destinés à la coloration directe des fibres kératiniques.

Or, la demanderesse vient maintenant de découvrir, de façon totalement inattendue et surprenante, que certaines nouvelles bases d'oxydation monobenzéniques de formule (I) définie ci-après comportant au moins un groupement cationique Z, Z étant choisi parmi des chaînes aliphatiques quaternisées et des chaînes aliphatiques contenant au moins un cycle saturé quaternisé, non seulement conviennent pour une utilisation comme précurseurs de colorant d'oxydation, mais en outre qu'elles permettent d'obtenir des compositions tinctoriales conduisant à des colorations puissantes couvrant une large palette de couleurs et présentant d'excellentes propriétés de résistances aux différents traitements que peuvent subir les fibres kératiniques. Enfin, ces composés s'avèrent être aisément synthétisables.

Ces découvertes sont à la base de la présente invention.

L'invention a donc pour premier objet de nouveaux composés de formule (I) suivante, et leurs sels d'addition avec un acide : 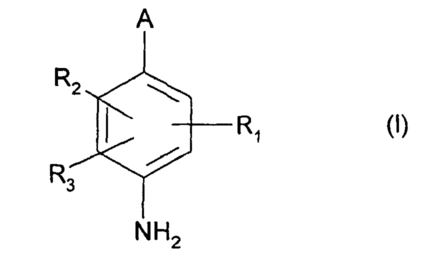 dans laquelle :
- R₁, R₂, R₃, qui peuvent être identiques ou différents, représentent un atome d'hydrogène ; un atome d'halogène ; un groupement Z ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) ; un radical carboxy ; un radical alkyl(C₁-C₆) carboxy; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) ; un radical N-Z-aminosulfonylalkyle(C₁-C₆) ; un radical N-alkyl(C₁-C₆)aminosulfonyl-alkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl-(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆)carboxy, carbamyle, N-alkyl(C₁-C₆)carbamyle, N,N-dialkyl(C₁-C₆)carbamyle, alkyl(C₁-C₆)sulfonyle, aminosulfonyle, N-Z-aminosulfonyle, N-alkyl(C₁-C₆)aminosulfonyle, N,N-dialkyl(C₁-C₆)aminosulfonyle, thiocarbamyle, formyle, ou par un groupement Z ; ou un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ;
- R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, et le groupement Z ;
- A représente un groupement -NR₄R₅ ou un radical hydroxyle ;
- R₄ et R₅, identiques ou différents, représentent un atome d'hydrogène ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ ; un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle ;
- Z représente un groupement de formule (II) suivante : 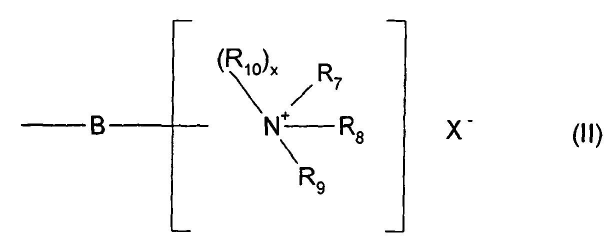
dans laquelle :
- B est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, pouvant être substituée par un ou plusieurs radicaux hydroxyle ou alcoxy en C₁-C₆, et pouvant porter une ou plusieurs fonctions cétone ;
- R₇, R₈ et R₉, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical carbamylalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₇, R₈ et R₉ peuvent également former ensemble, avec l'atome d'azote auquel ils sont rattachés, un cycle saturé à 5 ou 6 chaînons carboné ou contenant un ou plusieurs hétéroatomes tel que par exemple un cycle pyrrolidine, un cycle pipéridine, un cycle pipérazine ou un cycle morpholine, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)Silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
   l'un des radicaux R₇, R₈ et R₉ peut également représenter un second groupement Z identique ou différent du premier groupement Z ;
- X⁻ représente un anion monovalent ou divalent et est de préférence choisi parmi un atome d'halogène tel que le chlore, le brome, le fluor ou l'iode, un hydroxyde, un hydrogènesulfate, ou un alkyl(C₁-C₆)sulfate tel que par exemple un méthylsulfate ou un éthylsulfate ;
- R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle ; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁₋C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆ ;
- x est un nombre entier égal à 0 ou 1 ; avec les conditions suivantes :
   - lorsque x = 0, alors le bras de liaison B est rattaché à l'atome d'azote portant les radicaux R₇ à R₉ ;
      - lorsque x = 1, alors deux des radicaux R₇ à R₉ forment conjointement avec l'atome d'azote auquel ils sont rattachés un cycle saturé à 5 ou 6 chaînons tel que défini précédemment, et le bras de liaison B est porté par un atome de carbone dudit cycle saturé ;
      étant entendu :
   - que le nombre de groupements Z est au moins égal à 1.

Comme indiqué précédemment, les colorations obtenues avec la composition de teinture d'oxydation conforme à l'invention sont puissantes et couvrent une large palette de couleurs. Elles présentent de plus d'excellentes propriétés de résistance vis à vis de l'action des différents agents extérieurs (lumière, intempéries, lavage, ondulation permanente, transpiration, frottements).

Dans la formule (I) ci-dessus les radicaux alkyle et alcoxy peuvent être linéaires ou ramifiés.

Parmi les composés de formule (I) ci-dessus, on préfère plus particulièrement
- le chlorure de [2-(2,5-diamino-phénoxy)-éthyl]-diéthyl-méthyl-ammonium ;
- le chlorure de 1-{[5-amino-2-(2-hydroxyéthylamino)-phénylcarbamoyl]-méthyl}-1,4-diméthyl-pipérazin-1-ium et leurs sels d'addition avec un acide.

Les composés de formule (I) conformes à l'invention peuvent être facilement obtenus, selon des méthodes bien connues de l'état de la technique :
- soit par réduction des composés nitrés cationiques correspondants (para-nitranilines cationiques ou para-nitrophénols cationiques),
- soit par réduction des composés nitrosés cationiques correspondants (obtenus par exemple par nitrosation d'une aniline tertiaire ou d'un phénol correspondant),
- soit par réduction des composés azoïques cationiques correspondants (coupure réductrice).

Cette étape de réduction (obtention d'une amine aromatique primaire) qui confère au composé synthétisé son caractère de composé oxydable (de base d'oxydation) suivie ou non d'une salification, est en général, par commodité, la dernière étape de la synthèse.

Cette réduction peut intervenir plus tôt dans la suite des réactions conduisant à la préparation des composés de formule (I), et selon des procédés bien connus il faut alors "protéger" l'amine primaire créée (par exemple par une étape d'acétylation, de benzènesulfonation, etc...), faire ensuite la ou les substitutions ou modifications désirées (y compris la quaternisation) et terminer par la "déprotection" (en général en milieu acide) de la fonction amine.

De même la fonction phénolique peut être protégée selon des procédés bien connus par un radical benzyle ("déprotection" par réduction catalytique) ou par un radical acétyle ou mésyle ("déprotection" en milieu acide).

Lorsque la synthèse est terminée, les composés de formule (I) conformes à l'invention peuvent, le cas échéant, être récupérés par des méthodes bien connues de l'état de la technique telles que la cristallisation ou la distillation.

Un autre objet de l'invention est l'utilisation des composés de formules (I) conformes à l'invention à titre de base d'oxydation pour la teinture d'oxydation des fibres kératiniques, et en particulier des fibres kératiniques humaines telles que les cheveux.

L'invention a également pour objet une composition pour la teinture d'oxydation des fibres kératiniques et en particulier des fibres kératiniques humaines telles que les cheveux, caractérisée par le fait qu'elle comprend à titre de base d'oxydation, dans un milieu approprié pour la teinture, au moins un composé de formule (I) conforme à l'invention.

Le ou les composés de formule (I) conformes à l'invention représentent de préférence de 0,0005 à 12 % en poids environ du poids total de la composition tinctoriale, et encore plus préférentiellement de 0,005 à 6 % en poids environ de ce poids.

Le milieu approprié pour la teinture (ou support) est généralement constitué par de l'eau ou par un mélange d'eau et d'au moins un solvant organique pour solubiliser les composés qui ne seraient pas suffisamment solubles dans l'eau. A titre de solvant organique, on peut par exemple citer les alcanols inférieurs en C₁-C₄, tels que l'éthanol et l'isopropanol ; le glycérol ; les glycols et éthers de glycols comme le 2-butoxyéthanol, le propylèneglycol, le monométhyléther de propylèneglycol, le monoéthyléther et le monométhyléther du diéthylèneglycol, ainsi que les alcools aromatiques comme l'alcool benzylique ou le phénoxyéthanol, les produits analogues et leurs mélanges.

Les solvants peuvent être présents dans des proportions de préférence comprises entre 1 et 40 % en poids environ par rapport au poids total de la composition tinctoriale, et encore plus préférentiellement entre 5 et 30 % en poids environ.

Le pH de la composition tinctoriale conforme à l'invention est généralement compris entre 3 et 12 environ, et de préférence entre 5 et 11 environ. Il peut être ajusté à la valeur désirée au moyen d'agents acidifiants ou alcalinisants habituellement utilisés en teinture des fibres kératiniques.

Parmi les agents acidifiants, on peut citer, à titre d'exemple, les acides minéraux ou organiques comme l'acide chlorhydrique, l'acide orthophosphorique, l'acide sulfurique, les acides carboxyliques comme l'acide acétique, l'acide tartrique, l'acide citrique, l'acide lactique, les acides sulfoniques.

Parmi les agents alcalinisants on peut citer, à titre d'exemple, l'ammoniaque, les carbonates alcalins, les alcanolamines telles que les mono-, di- et triéthanolamines ainsi que leurs dérivés, les hydroxydes de sodium ou de potassium et les composés de formule (III): 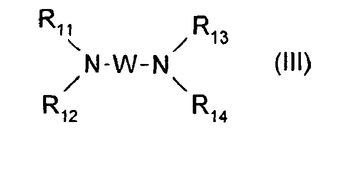 dans laquelle W est un reste propylène éventuellement substitué par un groupement hydroxyle ou un radical alkyle en C₁-C₆ ; R₁₁, R₁₂, R₁₃ et R₁₄, identiques ou différents, représentent un atome d'hydrogène, un radical alkyle en C₁-C₆ ou hydroxyalkyle en C₁-C₆.
La composition tinctoriale conforme à l'invention peut encore contenir, en plus des colorants définis ci-dessus, au moins une base d'oxydation additionnelle qui peut être choisie parmi les bases d'oxydation classiquement utilisées en teinture d'oxydation et parmi lesquelles on peut notamment citer les paraphénylènediamines différentes des composés de formule (I) conformes à l'invention, les bis-phénylalkylènediamines, les para-aminophénols différents des composés de formule (I) conformes à l'invention, les ortho-aminophénols et les bases hétérocycliques.

Parmi les paraphénylènediamines, on peut plus particulièrement citer à titre d'exemple, la paraphénylènediamine, la paratoluylènediamine, la 2,6-diméthyl paraphénylènediamine, la 2-β-hydroxyéthyl paraphénylènediamine, la 2-n-propyl paraphénylènediamine, la 2-isopropyl paraphénylènediamine, la N-(β-hydroxypropyl) paraphénylènediamine, la N,N-bis-(β-hydroxyéthyl) paraphénylènediamine, la 4-amino N-(β-méthoxyéthyl) aniline, les paraphénylènediamines décrites dans la demande de brevet français FR 2 630 438, et leurs sels d'addition avec un acide.

Parmi les bis-phénylalkylènediamines, on peut plus particulièrement citer à titre d'exemple, le N,N'-bis-(β-hydroxyéthyl) N,N'-bis-(4'-aminophényl) 1,3-diamino propanol, la N,N'-bis-(β-hydroxyéthyl) N,N'-bis-(4'-aminophényl) éthylènediamine, la N,N'-bis-(4-aminophényl) tétraméthylènediamine, la N,N'-bis-(β-hydroxyéthyl) N,N'-bis-(4-aminophényl) tétraméthylènediamine, la N,N'-bis-(4-méthylaminophényl) tétraméthylènediamine, la N,N'-bis-(éthyl) N,N'-bis-(4'-amino, 3'-méthylphényl) éthylènediamine, et leurs sels d'addition avec un acide.

Parmi les para-aminophénols, on peut plus particulièrement citer à titre d'exemple, le para-aminophénol, le 4-amino 3-méthyl phénol, le 4-amino 3-fluoro phénol, le 4-amino 3-hydroxyméthyl phénol, le 4-amino 2-méthyl phénol, le 4-amino 2-hydroxyméthyl phénol, le 4-amino 2-méthoxyméthyl phénol, le 4-amino 2-aminométhyl phénol, le 4-amino 2-(β-hydroxyéthyl aminométhyl) phénol, et leurs sels d'addition avec un acide.
Parmi les ortho-aminophénols, on peut plus particulièrement citer à titre d'exemple, le 2-amino phénol, le 2-amino 5-méthyl phénol, le 2-amino 6-méthyl phénol, le 5-acétamido 2-amino phénol, et leurs sels d'addition avec un acide.

Parmi les bases hétérocycliques, on peut plus particulièrement citer à titre d'exemple, les dérivés pyridiniques, les dérivés pyrimidiniques et les dérivés pyrazoliques.

Lorsqu'elles sont utilisées, ces bases d'oxydation additionnelles représentent de préférence de 0,0005 à 12 % en poids environ du poids total de la composition tinctoriale, et encore plus préférentiellement de 0,005 à 6 % en poids environ de ce poids.

Les compositions de teinture d'oxydation conformes à l'invention peuvent également renfermer au moins un coupleur et/ou au moins un colorant direct, notamment pour modifier les nuances ou les enrichir en reflets.

Les coupleurs utilisables dans les compositions de teinture d'oxydation conformes à l'invention peuvent être choisis parmi les coupleurs utilisés de façon classique en teinture d'oxydation et parmi lesquels on peut notamment citer les métaphénylènediamines, les méta-aminophénols, les métadiphénols et les coupleurs hétérocycliques tels que par exemple les dérivés indoliques, les dérivés indoliniques, les dérivés pyridiniques et les pyrazolones, et leurs sels d'addition avec un acide.

Ces coupleurs sont plus particulièrement choisis parmi le 2-méthyl 5-amino phénol, le 5-N-(β-hydroxyéthyl)amino 2-méthyl phénol, le 3-amino phénol, le 1,3-dihydroxy benzène, le 1,3-dihydroxy 2-méthyl benzène, le 4-chloro 1,3-dihydroxy benzène, le 2,4-diamino 1-(β-hydroxyéthyloxy) benzène, le 2-amino 4-(β-hydroxyéthylamino) 1-méthoxy benzène, le 1,3-diamino benzène, le 1,3-bis-(2,4-diaminophénoxy) propane, le sésamol, l'α-naphtol, le 6-hydroxy indole, le 4-hydroxy indole, le 4-hydroxy N-méthyl indole, la 6-hydroxy indoline, la 6-hydroxy benzomorpholine, la 2,6-dihydroxy 4-méthyl pyridine, le 1-H 3-méthyl pyrazole 5-one, le 1-phényl 3-méthyl pyrazole 5-one, et leurs sels d'addition avec un acide.

Lorsqu'ils sont présents ces coupleurs représentent de préférence de 0,0001 à 10 % en poids environ du poids total de la composition tinctoriale et encore plus préférentiellement de 0,005 à 5 % en poids environ de ce poids.

D'une manière générale, les sels d'addition avec un acide utilisables dans le cadre des compositions tinctoriales de l'invention (composés de formule (I), bases d'oxydation additionnelles et coupleurs) sont notamment choisis parmi les chlorhydrates, les bromhydrates, les sulfates, les citrates, les succinates, les tartrates, les lactates et les acétates.

La composition tinctoriale conforme à l'invention peut également renfermer divers adjuvants utilisés classiquement dans les compositions pour la teinture des cheveux, tels que des agents tensio-actifs anioniques, cationiques, non-ioniques, amphotères, zwittérioniques ou leurs mélanges, des polymères anioniques, cationiques, non-ioniques, amphotères, zwittérioniques ou leurs mélanges, des agents épaississants minéraux ou organiques, des agents antioxydants, des agents de pénétration, des agents séquestrants, des parfums, des tampons, des agents dispersants, des agents de conditionnement tels que par exemple des silicones, des agents filmogènes, des agents conservateurs, des agents opacifiants.

Bien entendu, l'homme de l'art veillera à choisir ce ou ces éventuels composés complémentaires de manière telle que les propriétés avantageuses attachées intrinsèquement à la composition de teinture d'oxydation conforme à l'invention ne soient pas, ou substantiellement pas, altérées par la ou les adjonctions envisagées.

La composition tinctoriale selon l'invention peut se présenter sous des formes diverses, telles que sous forme de liquides, de crèmes, de gels, ou sous toute autre forme appropriée pour réaliser une teinture des fibres kératiniques, et notamment des cheveux humains.

L'invention a également pour objet un procédé de teinture des fibres kératiniques et en particulier des fibres kératiniques humaines telles que les cheveux mettant en oeuvre la composition tinctoriale telle que définie précédemment.

Selon ce procédé, on applique sur les fibres au moins une composition tinctoriale telle que définie précédemment, la couleur étant révélée à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

Selon une forme de mise en oeuvre préférée du procédé de teinture de l'invention, on mélange de préférence, au moment de l'emploi, la composition tinctoriale décrite ci-dessus avec une composition oxydante contenant, dans un milieu approprié pour la teinture, au moins un agent oxydant présent en une quantité suffisante pour développer une coloration. Le mélange obtenu est ensuite appliqué sur les fibres kératiniques et on laisse poser pendant 3 à 50 minutes environ, de préférence 5 à 30 minutes environ, après quoi on rince, on lave au shampooing, on rince à nouveau et on sèche.

L'agent oxydant peut être choisi parmi les agents oxydants classiquement utilisés pour la teinture d'oxydation des fibres kératiniques, et parmi lesquels on peut citer le peroxyde d'hydrogène, le peroxyde d'urée, les bromates de métaux alcalins, les persels tels que les perborates et persulfates. Le peroxyde d'hydrogène est particulièrement préféré.

Le pH de la composition oxydante renfermant l'agent oxydant tel que défini ci-dessus est tel qu'après mélange avec la composition tinctoriale, le pH de la composition résultante appliquée sur les fibres kératiniques varie de préférence entre 3 et 12 environ, et encore plus préférentiellement entre 5 et 11. Il est ajusté à la valeur désirée au moyen d'agents acidifiants ou alcalinisants habituellement utilisés en teinture des fibres kératiniques et tels que définis précédemment.

La composition oxydante telle que définie ci-dessus peut également renfermer divers adjuvants utilisés classiquement dans les compositions pour la teinture des cheveux et tels que définis précédemment.

La composition qui est finalement appliquée sur les fibres kératiniques peut se présenter sous des formes diverses, telles que sous forme de liquides, de crèmes, de gels, ou sous toute autre forme appropriée pour réaliser une teinture des fibres kératiniques, et notamment des cheveux humains.

Un autre objet de l'invention est un dispositif à plusieurs compartiments ou "kit" de teinture ou tout autre système de conditionnement à plusieurs compartiments dont un premier compartiment renferme la composition tinctoriale telle que définie ci-dessus et un second compartiment renferme la composition oxydante telle que définie ci-dessus. Ces dispositifs peuvent être équipés d'un moyen permettant de délivrer sur les cheveux le mélange souhaité, tel que les dispositifs décrits dans le brevet FR-2 586 913 au nom de la demanderesse.

Les exemples qui suivent sont destinés à illustrer l'invention sans pour autant en limiter la portée.

### EXEMPLES DE PREPARATION

### EXEMPLE DE PREPARATION 1 : Synthèse du monochlorure, dichlorhydrate, monohydrate de [2-(2,5-diamino-phénoxy)-éthyl]-diéthyl-méthyl-ammonium

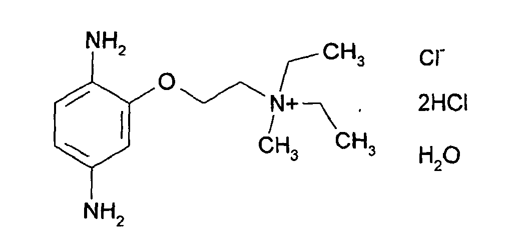

### a) Préparation du méthylsulfate de [2-(2-acétylamino-5-nitro-phénoxy)-éthyl]-diéthyl-méthyl-ammonium

La quaternisation de 59,1g (0,2 mole) de N-[2-(2-diéthylamino-éthoxy)-4-nitro-phényl]-acétamide dissous dans 700 ml d'acétate d'éthyle a été faite en ajoutant 20,9 ml (0,22 mole) de diméthylsulfate sous agitation, pendant une heure, à température ambiante.
Le composé quaternisé a précipité.

On a ensuite chauffé le mélange réactionnel pendant une heure à 50°C.
On a essoré les cristaux, réempaté dans le minimum d'éthanol absolu et séché à 50°C sous vide et sur anhydride phosphorique.

On a obtenu 71,3g de cristaux jaune pâle qui fondaient à 141-145°C (Kofler) et dont l'analyse élémentaire calculée pour C₁₆H₂₇N₃O₈S était :

| % | C | H | N | O | S |
|---|---|---|---|---|---|
| Calculé | 45,60 | 6,46 | 9,97 | 30,37 | 7,61 |
| Trouvé | 45,18 | 6,44 | 9,84 | 30,93 | 7,49 |

### b) Réduction du méthylsulfate de [2-(2-acétylamino-5-nitro-phénoxy)-éthyl]-diéthyl-méthyl-ammonium

On a chauffé au reflux de l'alcool un mélange de 100 ml d'éthanol à 96°, de 10 ml d'eau, de 50 g de zinc en poudre fine et de 1 g de chlorure d'ammonium.

On a ajouté par portions de façon à maintenir le reflux sans chauffage 42,1g (0,1 mole) de méthylsulfate de [2-(2-acétylamino-5-nitro-phénoxy)-éthyl]-diéthyl-méthyl-ammonium préparé à l'étape précédente. La réaction a été exothermique.

A la fin de l'addition on a maintenu le reflux pendant 10 minutes supplémentaires.

On a filtré bouillant et évaporé à sec sous pression réduite.
On a obtenu 44,4 g d'une huile jaune pâle de méthylsulfate de [2-(2-acétylamino-5-amino-phénoxy)-éthyl]-diéthyl-méthyl-ammonium.

### c) Désacétylation du méthylsulfate de [2-(2-acétylamino-5-amino-phénoxy)-éthyl]-diéthyl-méthyl-ammonium

Le méthylsulfate de [2-(2-acétylamino-5-amino-phénoxy)-éthyl]-diéthyl-méthyl-ammonium, obtenu à l'étape précédente (43,9 g), a été mis en solution, à température ambiante et sous agitation, dans 100 ml d'éthanol absolu chlorhydrique environ 5N.

Au bout d'une demi-heure un précipité cristallisé blanc est apparu.

La suspension a été chauffée une heure au reflux de l'alcool pour compléter l'échange d'anions.

On a refroidi, essoré, lavé à l'éthanol absolu et séché à 50°C sous vide et sur potasse.

On a obtenu 28,9g de cristaux blancs qui ont fondu avec décomposition à 110-120°C (Kofler) et dont l'analyse élémentaire calculée pour C₁₃H₂₆N₃OCl₃ + H₂O était :

| % | C | H | N | O | Cl |
|---|---|---|---|---|---|
| Calculé | 42,81 | 7,74 | 11,52 | 8,77 | 29,16 |
| Trouvé | 43,18 | 7,59 | 11,30 | 8,43 | 29,80 |

### EXEMPLE DE PREPARATION 2 : Synthèse du chlorure de 1-{[5-amino-2-(2-hydroxyéthylamino)-phénylcarbamoyl]-méthyl}-1,4-diméthyl-pipérazin-1-ium, trichlorhydrate

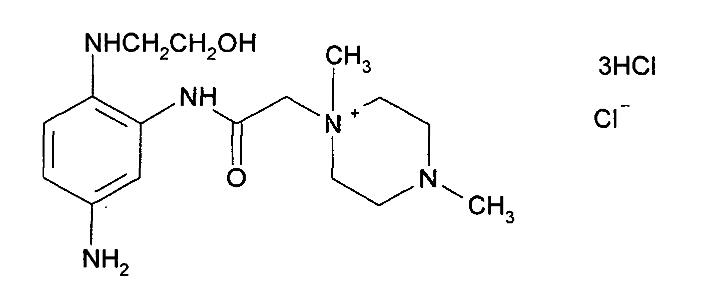

### a) Préparation du 2-chloro-N-[2-(2-hydroxyéthylamino)-5-nitro-phényl]-acétamide

On a mis en solution 82,5g (0,418 mole) de 2-(2-amino-4-nitro-phénylamino)-éthanol dans 400 ml de diméthylformamide et ajouté 34,6g (0,25 mole) de carbonate de potassium.

On a refroidi à 5°C et coulé goutte à goutte 34,7 ml (0,46 mole) de chlorure de chloracétyle en maintenant la température entre 5 et 12°C.

On a agité pendant une heure supplémentaire et versé le milieu réactionnel dans un mélange de 2 litres d'eau glacée et de 100 ml d'acide chlorhydrique à 36%.

Le précipité cristallisé a été essoré et purifié par recristallisation de l'acétonitrile au reflux.

On a obtenu 77,7 g de cristaux jaunes de 2-chloro-N-[2-(2-hydroxyéthylamino)-5-nitro-phényl]-acétamide qui ont fondu à 206°C (Kofler) et dont l'analyse élémentaire calculée pour C₁₀H₁₂N₃O₄Cl était :

| % | C | H | N | O | Cl |
|---|---|---|---|---|---|
| Calculé | 43,89 | 4,42 | 15,35 | 23,38 | 12,95 |
| Trouvé | 43,83 | 4,63 | 15,23 | 22,87 | 13,00 |

### b) Préparation du chlorure de 1-{[2-(2-hydroxvéthylamino)-5-nitro-phényl-carbamoyl]-méthyl}-1,4-diméthyl-pipérazin-1-ium

On a chauffé pendant 3 heures au reflux un mélange de 33,0 g (0,12 mole) de 2-chloro-N-[2-(2-hydroxyéthylamino)-5-nitro-phényl]-acétamide obtenu ci-dessus à l'étape précédente et de 27,4 g (0,24 mole) de 1,4-diméthyl-pipérazine dans 300 ml d'isobutanol.

On a laissé revenir le mélange à température ambiante, essoré le composé cristallisé et recristallise de l'éthanol à 96° au reflux.

On a obtenu 33,8 g de cristaux jaune clair de chlorure de 1-{[2-(2-hydroxyéthyl-amino)-5-nitro-phénylcarbamoyl]-méthyl}-1,4-diméthyl-pipérazin-1-ium qui ont fondu à plus de 260°C (Kofler) et dont l'analyse élémentaire était conforme à celle calculée pour C₁₆H₂₆N₅O₄Cl.

### c) Réduction du chlorure de 1-{[2-(2-hydroxyéthylamino)-5-nitro-phényl-carbamoyl]-méthyl}-1,4-diméthyl-pipérazin-1-ium

On a utilisé le mode opératoire décrit ci-dessus à l'exemple 2, étape c).

A partir de 33,0 g (0,085 mole) de chlorure de 1-{[2-(2-hydroxy-éthylamino)-5-nitro-phénylcarbamoyl]-méthyl}-1,4-diméthyl-piperazin-1-ium obtenu ci-dessus à l'étape précédente on a obtenu 29,3 g de cristaux blancs de chlorure de 1-{[5-amino-2-(2-hydroxyéthylamino)-phénylcarbamoyl]-méthyl}-1,4-diméthyl-pipérazin-1-ium, trichlorhydrate qui ont fondu avec décomposition à 250-260°C (Kofler) et dont la RMN 1H était conforme à celle du produit attendu

### EXEMPLES 1 à 5 DE TEINTURE EN MILIEU BASIQUE

On a préparé les compositions tinctoriales suivantes (teneurs en grammes) :

| **EXEMPLE** | **1** | **2** | **3** | **4** | **5** |
|---|---|---|---|---|---|
| Monochlorure, dichlorhydrate, monohydrate de [2-(2,5-Diamino-phénoxy)-éthyl]-diéthyl-méthyl-ammonium (composé de formule (I)) | 1,08 | 1,08 | 1,08 | 1,08 | 1,08 |
| Résorcine (Coupleur) | - | 0,33 | - | - | - |
| Méta-aminophénol (Coupleur) | - | - | 0,327 | - | - |
| 2-méthyl 5-N-(β-hydroxyéthyl)amino phénol (Coupleur) | - | - | - | 0,543 | - |
| Dichlorhydrate de 2,4-diamino-phénoxyéthanol (Coupleur) | - | - | - | - | 0,675 |
| Support de teinture commun | (*) | (*) | (*) | (*) | (*) |
| Eau déminéralisée q.s.p. | 100 g | 100 g | 100 g | 100 g | 100 g |

| | | | | | |
|---|---|---|---|---|---|
| (*) Support de teinture commun : - Ethanol à 96° 20 g - Sel pentasodique de l'acide diéthylène triamine pentacétique vendu sous la dénomination MASQUOL DTPA par la société PROTEX 1,08 g - Métabisulfite de sodium en solution aqueuse à 35 % de M.A. 0,58 g M.A. - Ammoniaque à 20 %10 g | | | | | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris, naturels ou permanentés, à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de TEINTURE** | **Nuance sur cheveux naturels** | **Nuance sur cheveux permanentés** |
|---|---|---|---|
| **1** | 10 ± 0,2 | Blond beige cendré irisé | Blond foncé cendré irisé |
| **2** | 10 ± 0,2 | Blond foncé nacré | Châtain irisé violine |
| **3** | 10 ± 0,2 | Violine cendré | Violine cendré |
| **4** | 10 ± 0,2 | Violine irisé | Violine irisé |
| **5** | 10 ± 0,2 | Bleu | Bleu |

### EXEMPLE 6 DE TEINTURE EN MILIEU BASIQUE

On a préparé les compositions tinctoriales suivantes (teneurs en grammes) :

| **EXEMPLE** | **6** |
|---|---|
| Chlorure de 1-{[5-amino-2-(2-hydroxyéthylamino)-phénylcarbamoyl]-méthyl}-1,4-diméthyl-pipérazin-1-ium, trichlorhydrate (composé de formule (I)) | 1,40 |
| 6-hydroxybenzomorpholine (Coupleur) | - |
| 1,3-dihydroxy 2-méthyl benzène (Coupleur) | |
| 3-amino phénol (Coupleur) | 0,327 |
| Support de teinture commun | (*) |
| Eau déminéralisée q.s.p. | 100 g |

| | |
|---|---|
| (*) Support de teinture commun : il est identique à celui utilisé pour les exemples de teinture 1 à 5 ci-dessus. | |

Au moment de l'emploi, on a mélangé poids pour poids chacune des compositions tinctoriales ci-dessus avec une solution de peroxyde d'hydrogène à 20 volumes (6 % en poids) de pH 3.

Le mélange obtenu a été appliqué sur des mèches de cheveux gris naturels à 90 % de blancs pendant 30 minutes. Les mèches ont ensuite été rincés, lavés avec un shampooing standard, rincées à nouveau puis séchées.

Les nuances obtenues figurent dans le tableau ci-après :

| **EXEMPLE** | **pH de teinture** | **Nuance obtenue** |
|---|---|---|
| **6** | 10 ± 0,2 | Acajou doré |

## Revendications

1. Composés de formule (I) suivante, et leurs sels d'addition avec un acide : 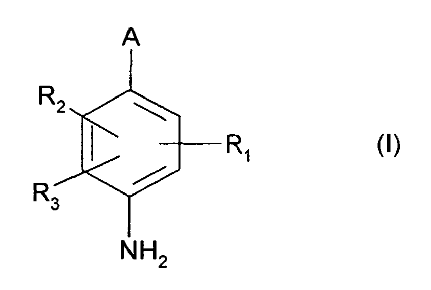 dans laquelle :
• R₁, R₂, R₃, qui peuvent être identiques ou différents, représentent un atome d'hydrogène ; un atome d'halogène ; un groupement Z ; un radical alkyl(C₁-C₆) carbonyle ; un radical aminoalkyl(C₁-C₆)carbonyle ; un radical N-Z-aminoalkyl(C₁-C₆)carbonyle ; un radical N-alkyl(C₁-C₆)aminoalkyl(C₁-C₆)carbonyle ; un radical N,N-dialkyl(C₁-C₆)aminoalkyl(C₁-C₆)carbonyle ; un radical aminoalkyl(C₁-C₆)carbonylalkyle(C₁-C₆) ; un radical N-Z-aminoalkyl(C₁-C₆)carbonylalkyle(C₁-C₆) ; un radical N-alkyl(C₁-C₆)aminoalkyl(C₁-C₆)carbonylalkyle(C₁-C₆) ; un radical N,N-dialkyl(C₁-C₆)aminoalkyl(C₁-C₆)carbonylalkyle(C₁-C₆) un radical carboxy ; un radical alkyl(C₁-C₆) carboxy; un radical alkyl(C₁-C₆) sulfonyle ; un radical aminosulfonyle ; un radical N-Z-aminosulfonyle ; un radical N-alkyl(C₁-C₆)aminosulfonyle ; un radical N,N-dialkyl(C₁-C₆)aminosulfonyle ; un radical aminosulfonylalkyle(C₁-C₆) un radical N-Z-aminosulfonylalkyle(C₁-C₆) ; un radical N-alkyl(C₁-C₆)aminosulfonyl-alkyle(C₁-C₆) ; un radical N,N-dialkyl(C₁-C₆)aminosulfonylalkyle(C₁-C₆) ; un radical carbamyle ; un radical N-alkyl(C₁-C₆)carbamyle ; un radical N,N-dialkyl-(C₁-C₆)carbamyle ; un radical carbamylalkyle(C₁-C₆) ; un radical N-alkyl(C₁-C₆)carbamylalkyle(C₁-C₆) ; un radical N,N-dialkyl(C₁-C₆)carbamylalkyle(C₁-C₆) ; un radical alkyle en C₁-C₆ ; un radical monohydroxyalkyle en C₁-C₆ un radical polyhydroxyalkyle en C₂-C₆ ; un radical alcoxy(C₁-C₆)alkyle en C₁-C₆ ; un radical trifluoroalkyle en C₁-C₆ ; un radical cyano ; un groupement OR₆ ou SR₆ ; un groupe amino protégé par un radical alkyl(C₁-C₆)carbonyle, alkyl(C₁-C₆)carboxy, trifluoroalkyl(C₁-C₆)carbonyle, aminoalkyl(C₁-C₆)carbonyle, N-Z-aminoalkyl(C₁-C₆)carbonyle, N-alkyl(C₁-C₆)aminoalkyl(C₁-C₆)carbonyle, N,N-dialkyl(C₁-C₆)aminoalkyl(C₁-C₆)carbonyle, alkyl(C₁-C₆)carboxy, carbamyle, N-alkyl(C₁-C₆)carbamyle, N,N-dialkyl(C₁-C₆)carbamyle, alkyl(C₁-C₆)sulfonyle, aminosulfonyle, N-Z-aminosulfonyle, N-alkyl(C₁-C₆)aminosulfonyle, N,N-dialkyl(C₁-C₆)aminosulfonyle, thiocarbamyle, formyle, ou par un groupement Z ; ou un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle, ou par un groupement Z ;
• R₆ désigne un radical alkyle en C₁-C₆ ; un radical monohydroxyalkyle en C₁-C₆ ; un radical polyhydroxyalkyle en C₂-C₆ ; un groupement Z ; un radical alcoxy(C₁-C₆)alkyle en C₁-C₆ ; un radical aryle ; un radical benzyle ; un radical carboxyalkyle en C₁-C₆ ; un radical alkyl(C₁-C₆)carboxyalkyle en C₁-C₆ ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical trifluoroalkyle en C₁-C₆ ; un radical aminosulfonylalkyle en C₁-C₆ ; un radical N-Z-aminosulfonylalkyle en C₁-C₆ un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)carbonylalkyle en C₁-C₆ ; un radical aminoalkyle en (C₁-C₆) ; un radical aminoalkyle en (C₁-C₆) dont l'amine est substituée par un ou deux radicaux identiques ou différents choisis parmi les radicaux alkyle(C₁-C₆), monohydroxyalkyle(C₁-C₆), polyhydroxyalkyle(C₂-C₆), alkyl(C₁-C₆)carbonyle, formyle, trifluoroalkyl(C₁-C₆)carbonyle, alkyl(C₁-C₆)carboxy, carbamyle, N-alkyl(C₁-C₆)carbamyle, N,N-dialkyl(C₁-C₆)carbamyle, thiocarbamyle, alkyl(C₁-C₆)sulfonyle, et le groupement Z ;
• A représente un groupement -NR₄R₅ ou un radical hydroxyle ;
• R₄ et R₅, identiques ou différents, représentent un atome d'hydrogène ; un radical alkyle en C₁-C₆ ; un radical monohydroxyalkyle en C₁-C₆ ; un radical polyhydroxyalkyle en C₂-C₆ ; un radical alcoxy(C₁-C₆)alkyle en C₁-C₆ ; un radical aryle ; un radical benzyle ; un radical cyanoalkyle en C₁-C₆ ; un radical carbamylalkyle en C₁-C₆ ; un radical N-alkyl(C₁-C₆)carbamylalkyle en C₁-C₆ ; un radical N,N-dialkyl(C₁-C₆)carbamylalkyle en C₁-C₆ ; un radical thiocarbamylalkyle en C₁-C₆ ; un radical trifluoroalkyle en C₁-C₆ un radical sulfoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical aminosulfonylalkyle en C₁-C₆ ; un radical N-alkyl(C₁-C₆)aminosulfonylalkyle en C₁-C₆ ; un radical N,N-dialkyl(C₁-C₆)aminosulfonylalkyle en C₁-C₆ ; un radical alkyl(C₁-C₆)carbonylalkyle en C₁-C₆ ; un radical aminoalkyle en C₁-C₆ ; un radical aminoalkyle en C₁-C₆ dont l'amine est substituée par un ou deux radicaux identiques ou différents choisis parmi les radicaux alkyle en C₁-C₆, monohydroxyalkyle en C₁-C₆, polyhydroxyalkyle en C₂-C₆, alkyl(C₁-C₆)carbonyle, carbamyle, N-alkyl(C₁-C₆)carbamyle ou N,N-dialkyl(C₁-C₆)carbamyle, alkyl(C₁-C₆)sulfonyle, formyle, trifluoroalkyl(C₁-C₆)carbonyle, alkyl(C₁-C₆)carboxy, thiocarbamyle ;
• Z représente un groupement de formule (II) suivante : 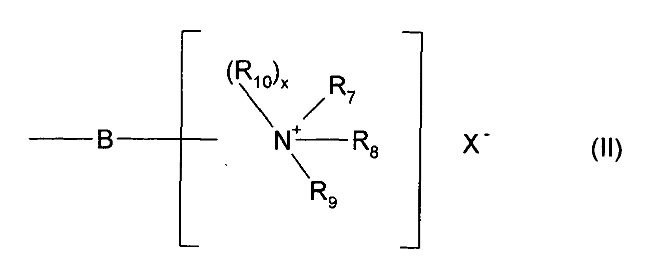
dans laquelle :
• B est un bras de liaison qui représente une chaîne alkyle comportant de préférence de 1 à 14 atomes de carbone, linéaire ou ramifiée pouvant être interrompue par un ou plusieurs hétéroatomes tels que des atomes d'oxygène, de soufre ou d'azote, pouvant être substituée par un ou plusieurs radicaux hydroxyle ou alcoxy en C₁-C₆, et pouvant porter une ou plusieurs fonctions cétone ;
• R₇, R₈ et R₉, identiques ou différents, représentent un radical alkyle en C₁-C₆, un radical monohydroxyalkyle en C₁-C₆, un radical polyhydroxyalkyle en C₂-C₆, un radical alcoxy(C₁-C₆)alkyle en C₁-C₆, un radical cyanoalkyle en C₁-C₆, un radical aryle, un radical benzyle, un radical carbamylalkyle en C₁-C₆, un radical trialkyl(C₁-C₆)silanealkyle en C₁-C₆ ou un radical aminoalkyle en C₁-C₆ dont l'amine est protégée par un radical alkyl(C₁-C₆)carbonyle, carbamyle, ou alkyl(C₁-C₆)sulfonyle ; deux des radicaux R₇, R₈ et R₉ peuvent également former ensemble, avec l'atome d'azote auquel ils sont rattachés, un cycle saturé à 5 ou 6 chaînons carboné ou contenant un ou plusieurs hétéroatomes, ledit cycle pouvant être ou non substitué par un atome d'halogène, un radical hydroxyle, un radical alkyle en C₁-C₆, un radical monohydroxyalkyle en C₁-C₆, un radical polyhydroxyalkyle en C₂-C₆, un radical nitro, un radical cyano, un radical cyanoalkyle en C₁-C₆, un radical alcoxy en C₁-C₆, un radical trialkyl(C₁-C₆)silanealkyle en C₁-C₆, un radical amido, un radical aldéhydo, un radical carboxyle, un radical alkylcarbonyle en C₁-C₆, un radical thio, un radical thioalkyle en C₁-C₆, un radical alkyl(C₁-C₆)thio, un radical amino, un radical amino protégé par un radical alkyl(C₁-C₆)carbonyle, carbamyle ou alkyl(C₁-C₆)sulfonyle ;
l'un des radicaux R₇, R₈ et R₉ peut également représenter un second groupement Z identique ou différent du premier groupement Z ;
• X⁻ représente un anion monovalent ou divalent ;
• R₁₀ représente un radical alkyle en C₁-C₆ ; un radical monohydroxyalkyle en C₁-C₆ ; un radical polyhydroxyalkyle en C₂-C₆ ; un radical aryle ; un radical benzyle ; un radical aminoalkyle en C₁-C₆, un radical aminoalkyle en C₁-C₆ dont l'amine est protégée par un radical alkyl(C₁-C₆)carbonyle, carbamyle ou alkyl(C₁-C₆)sulfonyle; un radical carboxyalkyle en C₁-C₆ ; un radical cyanoalkyle en C₁-C₆ ; un radical carbamylalkyle en C₁-C₆ ; un radical trifluoroalkyle en C₁-C₆ ; un radical trialkyl(C₁-C₆)silanealkyle en C₁-C₆ ; un radical sulfonamidoalkyle en C₁-C₆ ; un radical alkyl(C₁-C₆)carboxyalkyle en C₁-C₆ ; un radical alkyl(C₁-C₆)sulfinylalkyle en C₁-C₆ ; un radical alkyl(C₁-C₆)sulfonylalkyle en C₁-C₆ ; un radical alkyl(C₁-C₆)cétoalkyle en C₁-C₆ ; un radical N-alkyl(C₁-C₆)carbamylalkyle en C₁-C₆ ; un radical N-alkyl(C₁-C₆)sulfonamidoalkyle en C₁-C₆
• x est un nombre entier égal à 0 ou 1 ; avec les conditions suivantes :
- lorsque x = 0, alors le bras de liaison B est rattaché à l'atome d'azote portant les radicaux R₇ à R₉ ;
- lorsque x = 1, alors deux des radicaux R₇ à R₉ forment conjointement avec l'atome d'azote auquel ils sont rattachés un cycle saturé à 5 ou 6 chaînons tel que défini précédemment, et le bras de liaison B est porté par un atome de carbone dudit cycle saturé ;
étant entendu :
- que le nombre de groupements Z est au moins égal à 1.

2. Composés selon la revendication 1, **caractérisés par le fait que** X⁻ représente un atome d'halogène tel que le chlore, le brome, le fluor ou l'iode, un hydroxyde, un hydrogènesulfate, ou un alkyl(C₁-C₆)sulfate.

3. Composés selon la revendication 1 ou 2, **caractérisés par le fait qu'**ils sont choisis parmi :
- le chlorure de [2-(2,5-diamino-phénoxy)-éthyl]-diéthyl-méthyl-ammonium monohydrate ;
- le chlorure de 1-{[5-amino-2-(2-hydroxyéthylamino)-phénylcarbamoyl]-méthyl}-1,4-diméthyl-pipérazin-1-ium ;
et leurs sels d'addition avec un acide.

4. Utilisation des composés de formule (I) tels que définis à l'une quelconque des revendications 1 à 3, à titre de base d'oxydation pour la teinture d'oxydation des fibres kératiniques, et en particulier des fibres kératiniques humaines telles que les cheveux.

5. Composition pour la teinture d'oxydation des fibres kératiniques et en particulier des fibres kératiniques humaines telles que les cheveux, **caractérisée par le fait qu'**elle comprend, dans un milieu approprié pour la teinture, au moins un composé de formule (I) tel que défini à l'une quelconque des revendications 1 à 3, à titre de base d'oxydation.

6. Composition selon la revendication 5, **caractérisée par le fait que** le ou les composés de formule (I) représentent de 0,0005 à 12 % en poids du poids total de la composition tinctoriale.

7. Composition selon la revendication 6, **caractérisée par le fait que** le ou les composés de formule (I) représentent de 0,005 à 6 % en poids du poids total de la composition tinctoriale.

8. Composition selon l'une quelconque des revendications 5 à 7, **caractérisée par le fait que** le milieu approprié pour la teinture (ou support) est constitué par de l'eau ou par un mélange d'eau et d'au moins un solvant organique choisi parmi les alcanols inférieurs en C₁-C₄, le glycérol, les glycols et éthers de glycols, les alcools aromatiques, les produits analogues et leurs mélanges.

9. Composition selon l'une quelconque des revendications 5 à 8, **caractérisée par le fait qu'**elle présente un pH compris entre 3 et 12.

10. Composition selon l'une quelconque des revendications 5 à 9, **caractérisée par le fait qu'**elle renferme au moins une base d'oxydation additionnelle choisie parmi les paraphénylènediamines différentes des composés de formule (I), les bis-phénylalkylènediamines, les para-aminophénols différents des composés de formule (I), les ortho-aminophénols et les bases hétérocycliques.

11. Composition selon la revendication 10, **caractérisée par le fait que** la ou les bases d'oxydation additionnelles représentent de 0,0005 à 12 % en poids du poids total de la composition tinctoriale.

12. Composition selon l'une quelconque des revendications 6 à 12, **caractérisée par le fait qu'**elle renferme au moins un coupleur et/ou au moins un colorant direct.

13. Composition selon la revendication 12, **caractérisée par le fait que** le ou les coupleurs sont choisis parmi les métaphénylènediamines, les méta-aminophénols, les métadiphénols et les coupleurs hétérocycliques, et leurs sels d'addition avec un acide.

14. Composition selon la revendication 12 ou 13, **caractérisée par le fait que** le ou les coupleurs représentent de 0,0001 à 10 % en poids du poids total de la composition tinctoriale.

15. Composition selon l'une quelconque des revendications 5 à 14, **caractérisée par le fait que** les sels d'addition avec un acide sont choisis parmi les chlorhydrates, les bromhydrates, les sulfates, les citrates, les succinates, les tartrates, les lactates et les acétates.

16. Procédé de teinture des fibres kératiniques et en particulier des fibres kératiniques humaines telles que les cheveux **caractérisé par le fait que** l'on applique sur ces fibres au moins une composition tinctoriale telle que définie à l'une quelconque des revendications 5 à 15, et que l'on révèle la couleur à pH acide, neutre ou alcalin à l'aide d'un agent oxydant qui est ajouté juste au moment de l'emploi à la composition tinctoriale ou qui est présent dans une composition oxydante appliquée simultanément ou séquentiellement de façon séparée.

17. Procédé selon la revendication 16, **caractérisé par le fait que** l'agent oxydant est choisi parmi le peroxyde d'hydrogène, le peroxyde d'urée, les bromates de métaux alcalins, les persels tels que les perborates et persulfates.

18. Dispositif à plusieurs compartiments, ou "kit" de teinture à plusieurs compartiments, dont un premier compartiment renferme une composition tinctoriale telle que définie à l'une quelconque des revendications 5 à15 et un second compartiment renferme une composition oxydante.

## Claims

1. Compounds of following formula (I) and their addition salts with an acid: 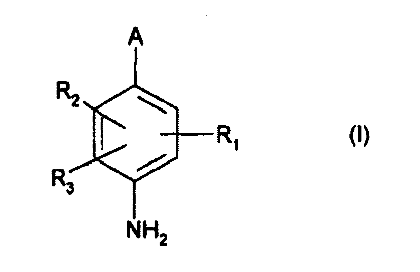 in which:
• R₁, R₂ and R₃, which can be identical or different, represent a hydrogen atom; a halogen atom; a Z group; a (C₁-C₆)alkylcarbonyl radical; an amino(C₁-C₆)alkylcarbonyl radical; an N-Z-amino(C₁-C₆)alkylcarbonyl radical; an N- (C₁-C₆) alkylamino (C₁-C₆) alkylcarbonyl radical; an N,N-di[(C₁-C₆)alkyl]amino(C₁-C₆)alkylcarbonyl radical; an amino (C₁-C₆) alkylcarbonyl (C₁-C₆) alkyl radical; an N-Z-amino (C₁-C₆) alkylcarbonyl (C₁-C₆) - alkyl radical; an N- (C₁-C₆) alkylamino (C₁-C₆) - alkylcarbonyl(C₁-C₆)alkyl radical; an N,N-di[(C₁-C₆)alkyl]amino(C₁-C₆)alkylcarbonyl(C₁-C₆)- alkyl radical; a carboxyl radical; a (C₁-C₆)-alkylcarboxyl radical; a (C₁-C₆)alkylsulphonyl radical; an aminosulphonyl radical; an N-Z-aminosulphonyl radical; an N-(C₁-C₆)alkylaminosulphonyl radical; an N,N-di[(C₁-C₆)alkyl]-aminosulphonyl radical; an aminosulphonyl-(C₁-C₆)alkyl radical; an N-Z-aminosulphonyl-(C₁-C₆)alkyl radical; an N- (C₁-C₆) alkylaminosulphonyl(C₁-C₆) alkyl radical; an N,N-di[(C₁-C₆)-alkyl]aminosulphonyl(C₁-C₆)alkyl radical; a carbamoyl radical; an N-(C₁-C₆)alkylcarbamoyl radical; an N,N-di[(C₁-C₆)alkyl]carbamoyl radical; a carbamoyl(C₁-C₆)alkyl radical; an N-(C₁-C₆)alkylcarbamoyl(C₁-C₆)alkyl radical; an N,N-di[(C₁-C₆)alkyl]carbamoyl(C₁-C₆)alkyl radical; a C₁-C₆ alkyl radical; a C₁-C₆ monohydroxyalkyl radical; a C₂-C₆ polyhydroxyalkyl radical; a (C₁-C₆)alkoxy(C₁-C₆)alkyl radical; a C₁-C₆ trifluoroalkyl radical; a cyano radical; an OR₆ or SR₆ group; an amino group protected by a (C₁-C₆) alkylcarbonyl, (C₁-C₆) alkylcarboxyl, trifluoro(C₁-C₆)alkylcarbonyl, amino (C₁-C₆)alkylcarbonyl, N-Z-amino(C₁-C₆)alkylcarbonyl, N-(C₁-C₆)alkylamino(C₁-C₆)alkylcarbonyl, N,N-di-[(C₁-C₆)alkyl] amino (C₁-C₆)alkylcarbonyl, (C₁-C₆)-alkylcarboxyl, carbamoyl, N-(C₁-C₆)alkylcarbamoyl, N,N-di[(C₁-C₆)alkyl]carbamoyl, (C₁-C₆)alkylsulphonyl, aminosulphonyl, N-Z-aminosulphonyl, N-(C₁-C₆)alkylaminosulphonyl, N,N-di[(C₁-C₆)alkyl]aminosulphonyl, thiocarbamoyl or formyl radical or by a Z group; or an amino (C₁-C₆) alkyl radical, the amine of which is substituted by one or two identical or different radicals chosen from C₁-C₆ alkyl, C₁-C₆ monohydroxyalkyl, C₂-C₆ polyhydroxyalkyl, (C₁-C₆)alkylcarbonyl, carbamoyl, N-(C₁-C₆) alkylcarbamoyl, N,N-di[(C₁-C₆)alkyl]-carbamoyl, (C₁-C₆)alkylsulphonyl, formyl, trifluoro(C₁-C₆)alkylcarbonyl, (C₁-C₆)alkylcarboxyl and thiocarbamoyl radicals or by a Z group;
• R₆ denotes a C₁-C₆ alkyl radical; a C₁-C₆ monohydroxyalkyl radical; a C₂-C₆ polyhydroxyalkyl radical; a Z group; a (C₁-C₆)alkoxy(C₁-C₆)alkyl radical; an aryl radical; a benzyl radical; a C₁-C₆ carboxyalkyl radical; a (C₁-C₆) alkyl-carboxy(C₁-C₆)alkyl radical; a C₁-C₆ cyanoalkyl radical; a C₁-C₆ carbamoylalkyl radical; an N-(C₁-C₆)alkylcarbamoyl(C₁-C₆)alkyl radical; an N,N-di[(C₁-C₆)alkyl]carbamoyl(C₁-C₆)alkyl radical; a C₁-C₆ trifluoroalkyl radical; a C₁-C₆ aminosulphonylalkyl radical; a C₁-C₆ N-Z-aminosulphonylalkyl radical; an N-(C₁-C₆)alkylaminosulphonyl(C₁-C₆)alkyl radical; an N,N-di[(C₁-C₆)-alkyl]aminosulphonyl(C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylcarbonyl(C₁-C₆)alkyl radical; a C₁-C₆ aminoalkyl radical; or a C₁-C₆ aminoalkyl radical, the amine of which is substituted by one or two identical or different radicals chosen from (C₁-C₆)alkyl, monohydroxy(C₁-C₆)alkyl, polyhydroxy(C₂-C₆)alkyl, (C₁-C₆)alkylcarbonyl, formyl, trifluoro (C₁-C₆) alkylcarbonyl, (C₁-C₆)-alkylcarboxyl, carbamoyl, N-(C₁-C₆)alkylcarbamoyl, N,N-di[(C₁-C₆)alkyl]carbamoyl, thiocarbamoyl and (C₁-C₆)alkylsulphonyl radicals and the Z group;
• A represents an -NR₄R₅ group or a hydroxyl radical;
• R₄ and R₅, which are identical or different, represent a hydrogen atom; a C₁-C₆ alkyl radical; a C₁-C₆ monohydroxyalkyl radical; a C₂-C₆ polyhydroxyalkyl radical; a (C₁-C₆)alkoxy-(C₁-C₆)alkyl radical; an aryl radical; a benzyl radical; a C₁-C₆ cyanoalkyl radical; a C₁-C₆ carbamoylalkyl radical; an N-(C₁-C₆)alkylcarbamoyl(C₁-C₆) alkyl radical; an N, N-di [(C₁-C₆)-alkyl] carbamoyl (C₁-C₆) alkyl radical; a C₁-C₆ thiocarbamoylalkyl radical; a C₁-C₆ trifluoroalkyl radical; a C₁-C₆ sulphoalkyl radical; a (C₁-C₆) alkylcarboxyl (C₁-C₆) alkyl radical; a (C₁-C₆) alkylsulphinyl (C₁-C₆) alkyl radical; a C₁-C₆ aminosulphonylalkyl radical; an N-(C₁-C₆)-alkylaminosulphonyl(C₁-C₆) alkyl radical; an N, N-di [(C₁-C₆) alkyl] aminosulphonyl (C₁-C₆) alkyl radical; a (C₁-C₆)alkylcarbonyl (C₁-C₆) alkyl radical; a C₁-C₆ aminoalkyl radical; or a C₁-C₆ aminoalkyl radical, the amine of which is substituted by one or two identical or different radicals chosen from C₁-C₆ alkyl, C₁-C₆ monohydroxyalkyl, C₂-C₆ polyhydroxyalkyl, (C₁-C₆)alkylcarbonyl, carbamoyl, N-(C₁-C₆)alkylcarbamoyl, N,N-di[(C₁-C₆)alkyl]carbamoyl, (C₁-C₆)alkylsulphonyl, formyl, trifluoro(C₁-C₆)-alkylcarbonyl, (C₁-C₆)alkylcarboxyl and thiocarbamoyl radicals;
• Z represents a group of following formula (II): 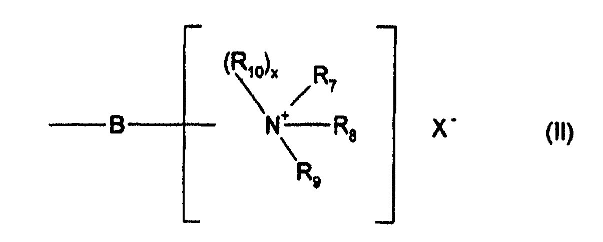
in which:
• B is a linking arm which represents a linear or branched alkylene chain, preferably comprising from 1 to 14 carbon atoms, which can be interrupted by one or more heteroatoms, such as oxygen, sulphur or nitrogen atoms, which can be substituted by one or more hydroxyl or C₁-C₆ alkoxy radicals and which can carry one or more ketone functional groups;
• R₇, R₈, and R₉, which are identical or different, represent a C₁-C₆ alkyl radical, a C₁-C₆ monohydroxyalkyl radical, a C₂-C₆ polyhydroxyalkyl radical, a (C₁-C₆)alkoxy(C₁-C₆)alkyl radical, a C₁-C₆ cyanoalkyl radical, an aryl radical, a benzyl radical, a C₁-C₆ carbamoylalkyl radical, a tri(C₁-C₆)alkylsilyl(C₁-C₆)alkyl radical or a C₁-C₆ aminoalkyl radical, the amine of which is protected by a (C₁-C₆)alkylcarbonyl, carbamoyl or (C₁-C₆)alkylsulphonyl radical; two of the R₇, R₈ and R₉ radicals can also together form, with the nitrogen atom to which they are attached, a saturated 5- or 6-membered carbon ring or one comprising one or more heteroatoms, it being possible for the said ring to be unsubstituted or substituted by a halogen atom, a hydroxyl radical, a C₁-C₆ alkyl radical, a C₁-C₆ monohydroxyalkyl radical, a C₂-C₆ polyhydroxyalkyl radical, a nitro radical, a cyano radical, a C₁-C₆ cyanoalkyl radical, a C₁-C₆ alkoxy radical, a tri (C₁-C₆) alkylsilyl (C₁-C₆) - alkyl radical, an amido radical, an aldehydo radical, a carboxyl radical, a C₁-C₆ alkylcarbonyl radical, a thio radical, a C₁-C₆ thioalkyl radical, a (C₁-C₆)alkylthio radical, an amino radical or an amino radical protected by a (C₁-C₆) alkylcarbonyl, carbamoyl or (C₁-C₆) - alkylsulphonyl radical;
one of the R₇, R₈ and R₉ radicals can also represent a second Z group identical to or different from the first Z group;
• X⁻ represents a monovalent or divalent anion;
• R₁₀ represents a C₁-C₆ alkyl radical; a C₁-C₆ monohydroxyalkyl radical; a C₂-C₆ polyhydroxyalkyl radical; an aryl radical; a benzyl radical; a C₁-C₆ aminoalkyl radical; a C₁-C₆ aminoalkyl radical, the amine of which is protected by a (C₁-C₆)alkylcarbonyl, carbamoyl or (C₁-C₆) alkylsulphonyl radical; a C₁-C₆ carboxyalkyl radical; a C₁-C₆ cyanoalkyl radical; a C₁-C₆ carbamoylalkyl radical; a C₁-C₆ trifluoroalkyl radical; a tri(C₁-C₆)alkylsilyl-(C₁-C₆)alkyl radical; a C₁-C₆ sulphonamidoalkyl radical; a (C₁-C₆)alkylcarboxyl(C₁-C₆)alkyl radical; a (C₁-C₆)alkylsulphinyl(C₁-C₆)alkyl radical; a (C₁-C₆)alkylsulphonyl(C₁-C₆)alkyl radical; a (C₁-C₆)alkylketo(C₁-C₆)alkyl radical; an N- (C₁-C₆) alkylcarbamoyl (C₁-C₆) alkyl radical; or an N- (C₁-C₆) alkylsulphonamido (C₁-C₆) alkyl radical;
• x is an integer equal to 0 or 1; with the following conditions:
- when x = 0, then the linking arm B is attached to the nitrogen atom carrying the R₇ to R₉ radicals;
- when x = 1, then two of the R₇ to R₉ radicals form, in conjunction with the nitrogen atom to which they are attached, a saturated 5- or 6-membered ring as defined above and the linking arm B is carried by a carbon atom of the said saturated ring;
it being understood:
- that the number of Z groups is at least equal to 1.

2. Compounds according to Claim 1, **characterized in that** X⁻ represents a halogen atom, such as chlorine, bromine, fluorine or iodine, a hydroxide, a hydrogensulphate or a (C₁-C₆)alkyl sulphate.

3. Compounds according to Claim 1 or 2, **characterized in that** they are chosen from:
- [2-(2,5-diaminophenoxy)ethyl]diethylmethyl-ammonium chloride monohydrate;
- 1-{[5-amino-2-(2-hydroxyethylamino)phenylcarbamoyl]methyl}-1,4-dimethylpiperazin-1-ium chloride;
and their addition salts with an acid.

4. Use of the compounds of formula (I) as defined in any one of Claims 1 to 3 as oxidation base for the oxidation dyeing of keratinous fibres, in particular of human keratinous fibres, such as the hair.

5. Composition for the oxidation dyeing of keratinous fibres, in particular human keratinous fibres, such as the hair, **characterized in that** it comprises, in a medium appropriate for dyeing, at least one compound of formula (I) as defined in any one of Claims 1 to 3 as oxidation base.

6. Composition according to Claim 5, **characterized in that** the compound or compounds of formula (I) represent from 0.0005 to 12% by weight of the total weight of the dyeing composition.

7. Composition according to Claim 6, **characterized in that** the compound or compounds of formula (I) represent from 0.005 to 6% by weight of the total weight of the dyeing composition.

8. Composition according to any one of Claims 5 to 7, **characterized in that** the medium appropriate for dyeing (or vehicle) is composed of water or of a mixture of water and at least one organic solvent chosen from lower C₁-C₄ alkanols, glycerol, glycols and glycol ethers, aromatic alcohols, the analogous products and their mixtures.

9. Composition according to any one of Claims 5 to 8, **characterized in that** it exhibits a pH of between 3 and 12.

10. Composition according to any one of Claims 5 to 9, **characterized in that** it includes at least one additional oxidation base chosen from para-phenylenediamines other than the compounds of formula (I), bisphenylalkylenediamines, para-aminophenols other than the compounds of formula (I), ortho-aminophenols and heterocyclic bases.

11. Composition according to Claim 10, **characterized in that** the additional oxidation base or bases represent from 0.0005 to 12% by weight of the total weight of the dyeing composition.

12. Composition according to any one of Claims 6 to 12, **characterized in that** it includes at least one coupler and/or at least one direct dye.

13. Composition according to Claim 12, **characterized in that** the coupler or couplers are chosen from meta-phenylenediamines, meta-aminophenols, metadiphenols and heterocyclic couplers, and their addition salts with an acid.

14. Composition according to Claim 12 or 13, **characterized in that** the coupler or couplers represent from 0.0001 to 10% by weight of the total weight of the dyeing composition.

15. Composition according to any one of Claims 5 to 14, **characterized in that** the addition salts with an acid are chosen from hydrochlorides, hydrobromides, sulphates, citrates, succinates, tartrates, lactates and acetates.

16. Method for dyeing keratinous fibres, in particular human keratinous fibres, such as the hair, **characterized in that** at least one dyeing composition as defined in any one of Claims 5 to 15 is applied to these fibres and **in that** the colour is developed at acidic, neutral or alkaline pH using an oxidizing agent which is added to the dyeing composition only at the time of use or which is present in an oxidizing composition applied simultaneously or sequentially in a separate manner.

17. Method according to Claim 16, **characterized in that** the oxidizing agent is chosen from hydrogen peroxide, urea hydrogen peroxide, alkali metal bromates, or persalts, such as perborates and persulphates.

18. Multicompartment device or multicompartment dyeing kit, a first compartment of which includes a dyeing composition as defined in any one of Claims 5 to 15 and a second compartment of which includes an oxidizing composition.

## Patentansprüche

1. Verbindungen der folgenden Formel (I) und ihre Additionssalze mit einer Säure: 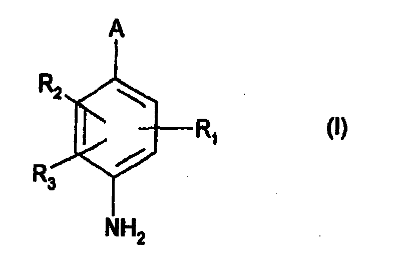 worin bedeuten:
· R₁, R₂ und R₃, die identisch oder voneinander verschieden sein können, ein Wasserstoffatom; ein Halogenatom; eine Gruppe Z; Alkyl(C₁₋₆)carbonyl; Aminoalkyl(C₁₋₆)carbonyl; N-Z-Aminoalkyl-(C₁₋₆)carbonyl; N-Alkyl(C₁₋₆)aminoalkyl(C₁₋₆)carbonyl; N,N-Dialkyl(C₁₋₆)aminoalkyl(C₁₋₆)carbonyl; Aminoalkyl(C₁₋₆)carbonyl-alkyl(C₁₋₆); N-Z-Aminoalkyl(C₁₋₆)carbonylalkyl(C₁₋₆); N-Alkyl(C₁₋₆)-aminoalkyl(C₁₋₆)carbonylalkyl(C₁₋₆); N,N-Dialkyl(C₁₋₆)aminoalkyl(C₁₋₆)carbonylalkyl(C₁₋₆); Carboxy; Alkyl(C₁₋₆)carboxy; Alkyl-(C₁₋₆)sulfonyl; Aminosulfonyl; N-Z-Aminosulfonyl; N-Alkyl(C₁₋₆)-aminosulfonyl; N,N-Dialkyl(C₁₋₆)aminosulfonyl; Aminosulfonyl-alkyl(C₁₋₆); N-Z-Aminosulfonylalkyl(C₁₋₆); N-Alkyl(C₁₋₆)amino-sulfonylalkyl(C₁₋₆); N,N-Dialkyl(C₁₋₆)aminosulfonylalkyl(C₁₋₆); Carbamoyl; N-Alkyl(C₁₋₆)carbamoyl; N,N-Dialkyl(C₁₋₆)carbamoyl; Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); Alkyl(C₁₋₆); Monohydroxyalkyl(C₁₋₆); Polyhydroxyalkyl(C₂₋₆); Alkoxy(C₁₋₆)alkyl(C₁₋₆); Trifluor-alkyl(C₁₋₆); Cyano; eine Gruppe OR₆ oder SR₆; eine Aminogruppe, die geschützt ist mit Alkyl(C₁₋₆)carbonyl; Alkyl(C₁₋₆)carboxy; Trifluoralkyl(C₁₋₆)carbonyl; Aminoalkyl(C₁₋₆)carbonyl; N-Z-Aminoalkyl(C₁₋₆)carbonyl; N-Alkyl(C₁₋₆)aminoalkyl(C₁₋₆)carbonyl; N,N-Dialkyl(C₁₋₆)aminoalkyl(C₁₋₆)carbonyl; Alkyl(C₁₋₆)carboxy; Carbamoyl; N-Alkyl(C₁₋₆)carbamoyl; N,N-Dialkyl(C₁₋₆)carbamoyl; Alkyl(C₁₋₆)sulfonyl; Aminosulfonyl; N-Z-Aminosulfonyl; N-Alkyl(C₁₋₆)aminosulfonyl; N,N-Dialkyl(C₁₋₆)aminosulfonyl; Thiocarbamoyl; Formyl oder mit einer Gruppe Z; oder eine Gruppe Aminoalkyl(C₁₋₆), deren Aminogruppe mit einer oder mit zwei Gruppen substituiert ist, die gleich oder verschieden sind und ausgewählt sind unter: Alkyl(C₁₋₆), Monohydroxyalkyl(C₁₋₆), Polyhydroxyalkyl(C₂-₆), Alkyl(C₁₋₆)carbonyl, Carbamoyl, N-Alkyl(C₁₋₆)carbamoyl, N,N-Dialkyl(C₁₋₆)carbamoyl, Alkyl(C₁₋₆)sulfonyl, Formyl, Trifluoralkyl(C₁₋₆)carbonyl, Alkyl(C₁₋₆)carboxy, Thiocarbamoyl oder mit einer Gruppe Z;
· R₆ Alkyl(C₁₋₆); Monohydroxyalkyl(C₁₋₆); Polyhydroxyalkyl(C₂₋₆); eine Gruppe Z; Alkoxy(C₁₋₆)alkyl(C₁₋₆); Aryl; Benzyl; Carboxyalkyl(C₁₋₆); Alkyl(C₁₋₆)carboxyalkyl(C₁₋₆); Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); Trifluoralkyl(C₁₋₆); Aminosul-fonylalkyl(C₁₋₆); N-Z-Aminosulfonylalkyl(C₁₋₆); N-Alkyl(C₁₋₆)-aminosulfonylalkyl(C₁₋₆); N,N-Dialkyl(C₁₋₆)aminosulfonyl-alkyl(C₁₋₆); Alkyl(C₁₋₆)sulfinylalkyl(C₁₋₆) ; Alkyl(C₁₋₆)sulfonyl-alkyl(C₁₋₆); Alkyl(C₁₋₆)carbonylalkyl(C₁₋₆); Aminoalkyl(C₁₋₆); eine Gruppe Aminoalkyl(C₁₋₆), deren Aminogruppe mit einer oder mit zwei Gruppen substituiert ist, die gleich oder verschieden sind und ausgewählt sind unter Alkyl(C₁₋₆), Monohydroxyalkyl(C₁₋₆), Polyhydroxyalkyl(C₂₋₆), Alkyl(C₁₋₆)carbonyl, Formyl, Trifluoralkyl(C₁₋₆)carbonyl, Alkyl(C₁₋₆)carboxy, Carbamoyl, N-Alkyl(C₁₋₆)-carbamoyl, N,N-Dialkyl(C₁₋₆)carbamoyl, Thiocarbamoyl, Alkyl(C₁₋₆)sulfonyl oder mit einer Gruppe Z;
· A eine Gruppe -NR₄R₅ oder Hydroxy;
· R₄ und R₅, die identisch oder voneinander verschieden sind, ein Wasserstoffatom; Alkyl(C₁₋₆); Monohydroxyalkyl(C₁₋₆); Polyhydroxyalkyl(C₂₋₆); Alkoxy(C₁₋₆)alkyl(C₁₋₆); Aryl; Benzyl; Cyanoalkyl(C₁₋₆); Carbamoylalkyl(C₁₋₆); N-Alkyl(C₁₋₆)carbamoylalkyl(C₁₋₆); N,N-Dialkyl(C₁₋₆)carbamoylalkyl(C₁₋₆); Thiocarbamoylalkyl(C₁₋₆); Trifluoralkyl(C₁₋₆); Sulfoalkyl(C₁₋₆); Alkyl(C₁₋₆)carboxyalkyl(C₁₋₆); Alkyl(C₁₋₆)sulfinylalkyl(C₁₋₆); Aminosulfonylalkyl(C₁₋₆); N-Alkyl-(C₁₋₆)aminosulfonylalkyl(C₁₋₆); N,N-Dialkyl(C₁₋₆)aminosulfonyl-alkyl(C₁₋₆); Alkyl(C₁₋₆)carbonylalkyl(C₁₋₆); Aminoalkyl(C₁₋₆); eine Gruppe Aminoalkyl(C₁₋₆), deren Aminogruppe mit einer oder mit zwei Gruppen substituiert ist, die gleich oder verschieden sind und ausgewählt sind unter Alkyl(C₁₋₆), Monohydroxyalkyl(C₁₋₆), Polyhydroxyalkyl(C₂₋₆), Alkyl(C₁₋₆)carbonyl, Carbamoyl, N-Alkyl(C₁₋₆)carbamoyl, N,N-Dialkyl(C₁₋₆)carbamoyl, Alkyl(C₁₋₆)-sulfonyl, Formyl, Trifluoralkyl(C₁₋₆)carbonyl, Alkyl(C₁₋₆)carboxy, Thiocarbamoyl;
· Z eine Gruppe der folgenden Formel (II): 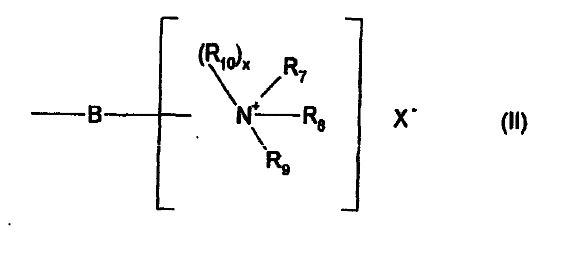
worin:
· B ist eine Verbindungsgruppe, die eine Alkylkette mit vorzugsweise 1 bis 14 Kohlenstoffatomen bedeutet, die geradkettig oder verzweigt vorliegt, durch ein oder mehrere Heteroatome, wie Sauerstoff, Schwefel oder Stickstoff, unterbrochen sein kann, mit einer oder mehreren Hydroxygruppen oder C₁₋₆-Alkoxygruppen substituiert sein kann und eine oder mehrere Ketofunktionen tragen kann;
· R₇, R₈ und R₉, die gleich oder verschieden sind, bedeuten Alkyl(C₁₋₆); Monohydroxyalkyl(C₁₋₆); Polyhydroxyalkyl(C₂₋₆); Alkoxy(C₁₋₆)alkyl(C₁₋₆); Cyanoalkyl(C₁₋₆); Aryl; Benzyl; Carbamoylalkyl(C₁₋₆); Trialkyl(C₁₋₆)silanalkyl(C₁₋₆); oder eine Gruppe Aminoalkyl(C₁₋₆), deren Aminogruppe geschützt ist mit Alkyl(C₁₋₆)-carbonyl, Carbamoyl oder Alkyl(C₁₋₆)sulfonyl; wobei zwei der Gruppen R₇, R₈ und R₉ auch gemeinsam mit dem Stickstoffatom, an das sie gebunden sind, einen 5- oder 6-gliedrigen, gesättigten Ring bilden können, der auf Kohlenstoffatomen basiert oder ein oder mehrere Heteroatome enthält, wobei der Ring unsubstituiert vorliegen kann oder substituiert sein kann mit Halogen, Hydroxy, Alkyl(C₁₋₆), Monohydroxyalkyl(C₁₋₆), Polyhydroxyalkyl(C₂₋₆), Nitro, Cyano, Cyanoalkyl(C₁₋₆), Alkoxy(C₁₋₆), Trialkyl(C₁₋₆)silanalkyl(C₁₋₆), Amido, Aldehydo, Carboxy, Alkyl(C₁₋₆)carbonyl, Thio, Thioalkyl(C₁₋₆), Alkyl(C₁₋₆)thio, Amino, einer Aminogruppe, die mit Alkyl(C₁₋₆)-carbonyl, Carbamoyl oder Alkyl(C₁₋₆)sulfonyl geschützt ist; wobei eine der Gruppen R₇, R₈ oder R₉ eine weitere Gruppe Z bedeuten kann, die mit der ersten Gruppe Z identisch oder von ihr verschieden ist;
· X- bedeutet ein- oder zweiwertiges Anion;
· R₁₀ bedeutet Alkyl(C₁₋₆); Monohydroxyalkyl(C₁₋₆); Polyhydroxyalkyl(C₂₋₆); Aryl; Benzyl; Aminoalkyl(C₁₋₆); eine Gruppe Aminoalkyl(C₁₋₆), deren Aminogruppe geschützt ist mit Alkyl(C₁₋₆)-carbonyl, Carbamoyl oder Alkyl(C₁₋₆)sulfonyl; Carboxyalkyl(C₁₋₆); Cyanoalkyl(C₁₋₆); Carbamoylalkyl(C₁₋₆); Trifluoralkyl(C₁₋₆); Trialkyl(C₁₋₆)silanalkyl(C₁₋₆); Sulfonamidoalkyl(C₁₋₆); Alkyl(C₁₋₆)carboxyalkyl(C₁₋₆); Alkyl(C₁₋₆)sulfinylalkyl(C₁₋₆); Alkyl(C₁₋₆)sulfonylalkyl(C₁₋₆); Alkyl(C₁₋₆)ketoalkyl(C₁₋₆); N-Alkyl(C₁₋₆)-carbamoylalkyl(C₁₋₆); N-Alkyl(C₁₋₆)sulfonamidoalkyl(C₁₋₆);
x bedeutet 0 oder 1; mit den folgenden Maßgaben:
- die Verbindungsgruppe B ist an das Stickstoffatom gebunden, das die Gruppen R₇ bis R₉ trägt, wenn x = 0.
- wenn x = 1: zwei der Gruppen R₇ bis R₉ bilden gemeinsam mit dem Stickstoffatom, an das sie gebunden sind, einen 5- oder 6-gliedrigen gesättigten Ring, wie oben definiert, und die Verbindungsgruppe B wird von einem Kohlenstoffatom des gesättigten Rings getragen;
mit der Maßgabe, dass:
- die Anzahl der kationischen Gruppen Z mindestens 1 ist.

2. Verbindungen nach Anspruch 1, **dadurch gekennzeichnet, dass** X⁻ ein Halogenatom, wie Chlor, Brom, Fluor oder Iod, Hydroxid, Hydrogensulfat oder Alkyl(C₁₋₆)sulfat bedeutet.

3. Verbindungen nach Anspruch 1 oder 2, **dadurch gekennzeichnet, dass** sie ausgewählt sind unter:
- [2-(2,5-Diaminophenoxy)-ethyl]-diethyl-methyl-ammoniumchlorid-Monohydrat;
- 1-{[5-Amino-2-(2-hydroxyethylamino)-phenylcarbamoyl]-methyl}-1,4-dimethyl-piperazin-1-ium;
und ihren Additionssalzen mit einer Säure.

4. Verwendung von Verbindungen der Formel (I) nach einem der Ansprüche 1 bis 3 als Oxidationsbasen zum oxidativen Färben von Keratinfasern und insbesondere menschlichen Keratinfasern wie Haaren.

5. Zusammensetzung zum oxidativen Färben von Keratinfasern und insbesondere menschlichen Keratinfasern wie Haaren, **dadurch gekennzeichnet, dass** sie in einem zum Färben geeigneten Medium mindestens eine Verbindung der Formel (I) nach einem der Ansprüche 1 bis 3 als Oxidationsbase enthält.

6. Zusammensetzung nach Anspruch 5, **dadurch gekennzeichnet, dass** die Verbindung(en) der Formel (I) 0,0005 bis 12 Gew.-% des Gesamtgewichts der Farbmittelzusammensetzung ausmachen.

7. Zusammensetzung nach Anspruch 6, **dadurch gekennzeichnet, dass** die Verbindung(en) der Formel (I) 0,005 bis 6 Gew.-% des Gesamtgewichts der Farbmittelzusammensetzung ausmachen.

8. Zusammensetzung nach einem der Ansprüche 5 bis 7, **dadurch gekennzeichnet, dass** das zum Färben geeignete Medium (oder der Träger) aus Wasser oder einem Gemisch von Wasser und mindestens einem organischen Lösungsmittel besteht, das unter den niederen C₁₋₄-Alkanolen, Glycerin, Glycolen und Glycolethern, aromatischen Alkoholen, analogen Produkten und deren Gemischen ausgewählt ist.

9. Zusammensetzung nach einem der Ansprüche 5 bis 8, **dadurch gekennzeichnet, dass** sie eine pH-Wert im Bereich von 3 bis 12 aufweist.

10. Zusammensetzung nach einem der Ansprüche 5 bis 9, **dadurch gekennzeichnet, dass** sie mindestens eine ergänzende Oxidationsbase enthält, die unter den p-Phenylendiaminen, die von den Verbindungen der Formel (I) verschieden sind, Bisphenylalkylendiaminen, p-Aminophenolen, die von den Verbindungen der Formel (I) verschieden sind, o-Aminophenolen und heterocyclischen Basen ausgewählt ist.

11. Zusammensetzung nach Anspruch 10, **dadurch gekennzeichnet, dass** die ergänzende(n) Oxidationsbase(n) 0,0005 bis 12 Gew.-% des Gesamtgewichts der Farbmittelzusammensetzung ausmachen.

12. Zusammensetzung nach einem der Ansprüche 6 bis 12, **dadurch gekennzeichnet, dass** sie mindestens einen Kuppler und/oder mindestens einen Direktfarbstoff enthält.

13. Zusammensetzung nach Anspruch 12, **dadurch gekennzeichnet, dass** der oder die Kuppler unter den m-Phenylendiaminen, m-Aminophenolen, m-Dihydroxybenzolen und heterocyclischen Kupplern und deren Additionssalzen mit einer Säure ausgewählt sind.

14. Zusammensetzung nach Anspruch 12 oder 13, **dadurch gekennzeichnet, dass** der oder die Kuppler etwa 0,0001 bis 10 Gew.-% des Gesamtgewichts der Farbmittelzusammensetzung ausmachen.

15. Zusammensetzung nach einem der Ansprüche 5 bis 14, **dadurch gekennzeichnet, dass** die Additionssalze mit einer Säure unter den Hydrochloriden, Hydrobromiden, Sulfaten, Citraten, Succinaten, Tartraten, Lactaten und Acetaten ausgewählt sind.

16. Verfahren zum Färben von Keratinfasern und insbesondere menschlichen Keratinfasern, wie zum Färben der Haare, **dadurch gekennzeichnet, dass** auf die Fasern mindestens eine Farbmittelzusammensetzung nach einem der Ansprüche 5 bis 15 aufgebracht wird und die Farbe bei einem sauren, neutralen oder alkalischen pH-Wert mit einem Oxidationsmittel gebildet wird, das bei der Anwendung zu der Farbmittelzusammensetzung gegeben wird oder das in einer oxidierenden Zusammensetzung vorliegt, die gleichzeitig oder getrennt davon anschließend aufgebracht wird.

17. Verfahren nach Anspruch 16, **dadurch gekennzeichnet, dass** das Oxidationsmittel unter Wasserstoffperoxid, Harnstoffperoxid, Alkalimetallbromaten und Salzen von Persäuren, wie Perboraten und Persulfaten, ausgewählt ist.

18. Vorrichtung mit mehreren Abteilungen oder "Kit" zum Färben mit mehreren Abteilungen, wobei eine erste Abteilung eine Farbmittelzusammensetzung nach einem der Ansprüche 5 bis 15 und eine zweite Abteilung eine oxidierende Zusammensetzung enthält.
